# EUROPEAN PATENT SPECIFICATION

(11) **EP 4 281 042 B1**
(45) Date of publication and mention of the grant of the patent: **12.08.2026**
(21) Application number: 22701367.9
(22) Date of filing: 20.01.2022
(51) Int. Cl.: A61K 9/08, A61K 9/19, A61K 31/43, A61K 31/551, A61P 31/04

(54) **FIXED DOSAGE ANTIBIOTIC COMPOSITIONS**
ANTIBIOTISCHE ZUSAMMENSETZUNGEN MIT FESTER DOSIERUNG
COMPOSITIONS D'ANTIBIOTIQUES À DOSE FIXE

(30) Priority: 20.01.2021 US 202163139363 P
(43) Date of publication of application: 29.11.2023
(73) Proprietor: Entasis Therapeutics Limited, Altrincham Cheshire WA14 2DT (GB)
(72) Inventor: JAIN, Rajeev A., Framingham, Massachusetts 01701 (US); RONSHEIM, Matthew, Sudbury, Massachusetts 01776 (US); FOGLIATO, Giovanni, 20067 Tribiano (IT); RESEMINI, Dario, 20067 Tribiano (IT)
(74) Representative: Potter Clarkson
(86) International application number: PCT/EP2022/051267
(87) International publication number: WO 2022/157259

(56) References cited:
- WO-A1-2016/081452
- ANONYMOUS: "EMEA/660371/2020 - european Medicines agency decision P/0499/2020", 22 December 2020 (2020-12-22), pages 1 - 8, XP055915242, Retrieved from the Internet <URL:https://www.ema.europa.eu/en/documents/pip-decision/p/0499/2020-ema-decision-22-december-2020-agreement-paediatric-investigation-plan-granting-deferral/sulbactam-sul-dur-emea-002807-pip01-20_en.pdf> [retrieved on 20220425]
- SAGAN OLEXIY ET AL: "Pharmacokinetics and Tolerability of Intravenous Sulbactam-Durlobactam with Imipenem-Cilastatin in Hospitalized Adults with Complicated Urinary Tract Infections, Including Acute Pyelonephritis", ANTIMICROBIAL AGENTS AND CHEMOTHERAPY, vol. 64, no. 3, 21 February 2020 (2020-02-21), US, XP055915085, ISSN: 0066-4804, Retrieved from the Internet <URL:https://journals.asm.org/doi/pdf/10.1128/AAC.01506-19> DOI: 10.1128/AAC.01506-19
- RODVOLD KEITH A. ET AL: "Plasma and Intrapulmonary Concentrations of ETX2514 and Sulbactam following Intravenous Administration of ETX2514SUL to Healthy Adult Subjects", ANTIMICROBIAL AGENTS AND CHEMOTHERAPY, vol. 62, no. 11, 20 August 2018 (2018-08-20), US, XP055915096, ISSN: 0066-4804, Retrieved from the Internet <URL:https://journals.asm.org/doi/pdf/10.1128/AAC.01089-18> DOI: 10.1128/AAC.01089-18
- MORO L.: "ETX-2514. Broad-spectrum beta-lactamase inhibitor, Treatment of multidrug-resistant infections", DRUGS OF THE FUTURE, vol. 43, no. 8, 1 January 2018 (2018-01-01), ES, pages 555 - 563, XP055915134, ISSN: 0377-8282, Retrieved from the Internet <URL:https://journals.prous.com/journals/servlet/xmlxsl/dof/20184308/pdf/df430555.pdf?p_JournalId=2&p_refId=2824713&p_IsPs=N> DOI: 10.1358/dof.2018.043.08.2824713
- ANONYMOUS: "Lyophilization of Parenteral (7/93) | FDA", 11 November 2014 (2014-11-11), pages 1 - 22, XP055915548, Retrieved from the Internet <URL:https://www.fda.gov/inspections-compliance-enforcement-and-criminal-investigations/inspection-guides/lyophilization-parenteral-793> [retrieved on 20220426]
- ANONYMOUS: "Lyophilization: The Basics - Drug Discovery and Development", 7 March 2017 (2017-03-07), pages 1 - 10, XP055915554, Retrieved from the Internet <URL:https://www.drugdiscoverytrends.com/lyophilization-the-basics/> [retrieved on 20220426]

## Description

### RELATED APPLICATIONS

This application claims the benefit of priority to U.S. Provisional Application No. 63/139,363, filed January 20, 2021.

### BACKGROUND

*Acinetobacter baumannii* is a Gram-negative opportunistic pathogen, and is one of the most prevalent causes of nosocomial infections. *A. baumannii* is commonly multidrug-resistant (MDR), with rates between 50% and 60% in the United States and greater than 80% in parts of Europe and Asia. Serious infections caused by MDR *A*. *baumannii* isolates are associated with high rates of morbidity, and the rate of mortality may range up to 50% or higher.

Prior to β-lactamase-mediated resistance, sulbactam (SUL) was one of the few antibiotics of choice for the treatment of *A. baumannii* infections. Currently, the minimal treatment options still effective against *A*. *baumannii* infections have poor efficacy and tolerability, resulting in mortality rates approaching 50% for *A. baumannii* pneumonia and blood stream infections.

Durlobactam (DUR; also known as ETX2514) is a novel, diazabicyclooctenone β-lactamase inhibitor (BLI) that exhibits potent inhibition of class A, C, and D β-lactamases (see e.g., Nat Microbiol 2:17104. doi:10.1038/nmicrobiol.2017.104). In vitro, durlobactam exhibits intrinsic antibacterial activity against some Enterobacteriaceae but has no significant intrinsic activity against *A*. *baumannii* complex (ABC) isolates. Durlobactam restores the in vitro activity of sulbactam against members of *A. baumannii* and the combination of sulbactam with durlobactam (SUL-DUR) is under development for the treatment of *A. baumannii* infections, as well as other bacterial-related infections.

Durlobactam sodium is highly hydroscopic and has poor flow properties. As a consequence, processing and administering SUL-DUR combination therapies are not ideal. Treatment with SUL-DUR involves separately reconstituting two vials of durlobactam sodium salt sterile drug component in water for injection and one vial of sulbactam sodium salt sterile drug component in water for injection. Fixed aliquots from each of the three vials are then transferred into an intravenous fluid bag and the resulting combination is then administered to the patient as required. This onerous process not only enhances the likelihood of user error, but also leads to unnecessary chemical and medical waste. A fixed dose composition comprising SUL-DUR is therefore needed.

### SUMMARY

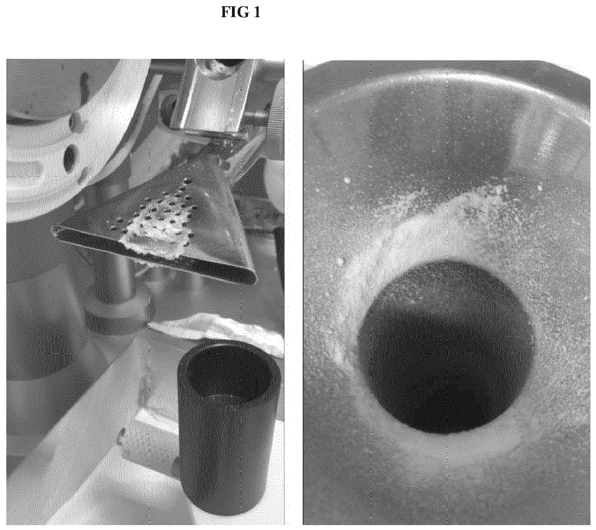

It has now been found that co-lyophilizing durlobactam sodium and sulbactam sodium together improves the flow properties of durlobactam sodium such that fixed dosage combinations of durlobactam-sulbactam are now possible. Initial attempts to formulate a fixed dosage comprised dry powder filling both DUR sodium salt and SUL sodium salt in glass vials. Due to the hygroscopic, poor flow properties of DUR sodium salt, significant powder adhesion to the parts of the filling equipment resulted (see **FIG. 1**). The drawback leads to unreliable and inconsistent product dosing. However, when DUR sodium salt and SUL sodium were co-lyophilized together, essentially no powder adhesion was observed. This result not only allows for fixed dosages combinations of durlobactam-sulbactam to now be attainable, but it was also surprising given the relatively difference between the calculated flowability parameters such as the Hausner ratio and compressibility index (aka Carr index), when compared to DUR sodium salt alone. See e.g., the Exemplification section below.

Disclosed herein, therefore, are co-lyophilized fixed dosage combinations of durlobactam or a pharmaceutically acceptable salt thereof and sulbactam or a pharmaceutically acceptable salt thereof. The disclosed fixed dosage combinations were also found to have good stability with almost no change in assay value and nominal impurity increase at 5 °C over 3 months. See e.g., **Table 3.** Similar results were observed at 25 °C/60% RH . See e.g., **Table 4.**

Also disclosed are pharmaceutically acceptable solutions comprising a co-lyophilized fixed dosage combination of durlobactam or a pharmaceutically acceptable salt thereof and sulbactam or a pharmaceutically acceptable salt thereof. Such solutions include, e.g., those in which a disclosed co-lyophilized fixed dosage combination is reconstituted in solvent for administration (e.g., water-for-injection).

Also disclosed herein are split-fill fixed dosage combinations of lyophilized durlobactam or a pharmaceutically acceptable salt thereof and sulbactam or a pharmaceutically acceptable salt thereof, wherein the sulbactam or salt thereof and the lyophilized durlobactam or salt thereof are filled sequentially.

Also disclosed is the use of a disclosed fixed dosage combination or reconstituted solution for treating a bacterial infection.

Also disclosed are packaged pharmaceutical kits comprising a disclosed fixed dosage combination or reconstituted solution.

Methods of making co-lyophilized fixed dosage combinations of SUL-DUR or reconstituted solutions thereof are further described.

### BRIEF DESCRIPTION OF THE FIGURES

**FIG 1****.** shows the DUR sodium salt powder residue observed from the centralized suction and on the feeding chute of an IMA MD300 filling machine during machinability trials.
**FIG 2****.** illustrates the appearance of DUR sodium salt powder following machine filling.
**FIG 3****.** shows the appearance of the bulk powder obtained from co-lyophilizing durlobactam and sulbactam.

### DETAILED DESCRIPTION

In one aspect, provided is a fixed dosage combination comprising durlobactam or a pharmaceutically acceptable salt thereof and sulbactam or a pharmaceutically acceptable salt thereof, wherein the durlobactam or a pharmaceutically acceptable salt thereof and the sulbactam or a pharmaceutically acceptable salt thereof are present as a co-lyophilized mixture.

Durlobactam and ETX2514 are synonymous and refer to the beta-lactamase inhibitor having the structure: 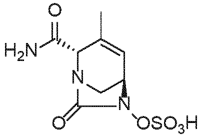 Durlobactam is disclosed in PCT/GB2013/050869 and PCT/US2015/061076. Durlobactam-Na, durlobactam-Na salt, DUR-Na, DUR-Na salt and the like are synonymous and refer to the sodium salt of durlobactam.

Sulbactam refers to the beta-lactamase inhibitor having the structure: 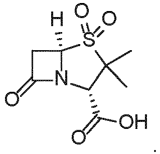 Unless otherwise specified, sulbactam includes the stereoisomer depicted above as well as all other possible stereoisomer and mixtures of stereoisomers. In one aspect, however, sulbactam as described herein refers to the depicted stereoisomer with a stereochemical enrichment or a molar excess of at least 60%, at least 65%, at least 70%, at least 80%, at least 85%, at least 90%, at least 95%, at least 97%, at least 98%, or at least 99%. Sulbactam-Na, sulbactam-Na salt, SUL-Na, SUL-Na salt and the like are synonymous and refer to the sodium salt of sulbactam.

When used as part of the claims and present disclosure, the term "consisting of" or "consists of' means that elements, steps, or ingredients not specified in the claim or description following the term are excluded, except for impurities ordinarily associated therewith. For example, a fixed dosage combination "consisting of" durlobactam or a pharmaceutically acceptable salt thereof together with and sulbactam or a pharmaceutically acceptable salt thereof means that the only elements present are durlobactam, sulbactam, or theirs salts, and impurities ordinarily associated therewith. The term "comprising", which is synonymous with "including," "containing," or "characterized by," is inclusive or open-ended and does not exclude additional, unrecited elements or method steps.

The terms "fixed dosage", "fixed dosage combination", and "fixed dosage composition", are used interchangeably and refer to two or more active pharmaceutical ingredients (such as DUR and SUL) comprised in a single dosage form e.g., in the same capsule, tablet, vial, suppository, etc.

SUL and DUR may form pharmaceutically acceptable acid or base salts, and in such cases administration of a compound as a salt may be appropriate. Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Examples of base salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, omithine, and so forth. In one aspect, the pharmaceutically acceptable salt for DUR or SUL, or both, comprised in a disclosed fixed dosage form is a potassium, lithium, calcium, or sodium salt. In one aspect, the pharmaceutically acceptable salt for DUR or SUL, or both, comprised in a disclosed fixed dosage form is a sodium salt.

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The fixed dosage combinations described herein comprise SUL and DUR in a co-lyophilized form. Lyophilization methods, also known as freeze-drying, are known in the art and are low temperature dehydration processes that involve freezing the product, lowering pressure, then removing the ice by sublimation. Co-lyophilization or co-lyophilized means that the SUL and DUR, or pharmaceutically acceptable salts of one or both, are lyophilized together (e.g., in the same vessel or container).

Also provided herein is a split-fill fixed dosage combination of lyophilized durlobactam or a pharmaceutically acceptable salt thereof and sulbactam or a pharmaceutically acceptable salt thereof, wherein the sulbactam or salt thereof and lyophilized durlobactam or salt thereof are filled sequentially.

Various amounts of SUL and DUR, or their salts may be used in a fixed dosage composition. In one alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions ranges from 0.1:2.0 w/w to 2.0:0.1 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions ranges from 0.2:1.9 w/w to 1.9:0.2 w/w, from 0.2:1.8 w/w to 1.8:0.2 w/w, from 0.3:1.7 w/w to 1.7:0.3 w/w, from 0.4:1.6 w/w to 1.6:0.4 w/w, from 0.5:1.5 w/w to 1.5:0.5 w/w, from 0.6:1.4 w/w to 1.4:0.6 w/w, from 0.7:1.3 w/w to 1.3:0.7 w/w, from 0.8:1.2 w/w to 1.2:0.8 w/w, or from 0.9:1.1 w/w to 1.1:0.9 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions ranges from 0.5:1.5 w/w to 1.5:0.5 w/w, from 0.7:1.3 w/w to 1.3:0.7 w/w, from 0.8:1.2 w/w to 1.2:0.8 w/w, or from 0.8:1.1 w/w to 1.1:0.8 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions ranges from 0.9:1.1 w/w to 1.1:0.9 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is 0.1:2.0 w/w, 0.1:1.9 w/w, 0.2:1.8 w/w, 0.3:1.7 w/w, 0.4:1.6 w/w, 0.5:1.5 w/w, 0.6:1.4 w/w, 0.7:1.3 w/w, 0.8:1.2 w/w, 0.9:1.1 w/w, 1:1 w/w, 1.1:0.9 w/w, 1.2:0.8 w/w, 1.3:0.7 w/w, 1.4:0.6 w/w, 1.5:0.5 w/w, 1.6:0.4 w/w, 1.7:0.3 w/w, 1.8:0.2 w/w, 1.9:0.1 w/w or 2.0:0.1 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is 1:1 w/w.

In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR and SUL ranging from 0.1:2.0 w/w to 2.0:0.1 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR and SUL ranging from 0.2:1.9 w/w to 1.9:0.2 w/w, from 0.2:1.8 w/w to 1.8:0.2 w/w, from 0.3:1.7 w/w to 1.7:0.3 w/w, from 0.4:1.6 w/w to 1.6:0.4 w/w, from 0.5:1.5 w/w to 1.5:0.5 w/w, from 0.6:1.4 w/w to 1.4:0.6 w/w, from 0.7:1.3 w/w to 1.3:0.7 w/w, from 0.8:1.2 w/w to 1.2:0.8 w/w, or from 0.9:1.1 w/w to 1.1:0.9 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR and SUL ranging from 0.5:1.5 w/w to 1.5:0.5 w/w, from 0.7:1.3 w/w to 1.3:0.7 w/w, from 0.8:1.2 w/w to 1.2:0.8 w/w, or from 0.8:1.1 w/w to 1.1:0.8 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR and SUL ranging from 0.9:1.1 w/w to 1.1:0.9 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR and SUL of 0.1:2.0 w/w, 0.1:1.9 w/w, 0.2:1.8 w/w, 0.3:1.7 w/w, 0.4:1.6 w/w, 0.5:1.5 w/w, 0.6:1.4 w/w, 0.7:1.3 w/w, 0.8:1.2 w/w, 0.9:1.1 w/w, 1:1 w/w, 1.1:0.9 w/w, 1.2:0.8 w/w, 1.3:0.7 w/w, 1.4:0.6 w/w, 1.5:0.5 w/w, 1.6:0.4 w/w, 1.7:0.3 w/w, 1.8:0.2 w/w, 1.9:0.1 w/w or 2.0:0.1 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR and SUL of 1:1 w/w.

In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR-Na Salt and SUL-Na Salt ranging from 0.1:2.0 w/w to 2.0:0.1 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR-Na Salt and SUL-Na Salt ranging from 0.2:1.9 w/w to 1.9:0.2 w/w, from 0.2:1.8 w/w to 1.8:0.2 w/w, from 0.3:1.7 w/w to 1.7:0.3 w/w, from 0.4:1.6 w/w to 1.6:0.4 w/w, from 0.5:1.5 w/w to 1.5:0.5 w/w, from 0.6:1.4 w/w to 1.4:0.6 w/w, from 0.7:1.3 w/w to 1.3:0.7 w/w, from 0.8:1.2 w/w to 1.2:0.8 w/w, or from 0.9:1.1 w/w to 1.1:0.9 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR-Na Salt and SUL-Na Salt ranging from 0.5:1.5 w/w to 1.5:0.5 w/w, from 0.7:1.3 w/w to 1.3:0.7 w/w, from 0.8:1.2 w/w to 1.2:0.8 w/w, or from 0.8:1.1 w/w to 1.1:0.8 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR-Na Salt and SUL-Na Salt ranging from 0.9:1.1 w/w to 1.1:0.9 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR-Na Salt and SUL-Na Salt of 0.1:2.0 w/w, 0.1:1.9 w/w, 0.2:1.8 w/w, 0.3:1.7 w/w, 0.4:1.6 w/w, 0.5:1.5 w/w, 0.6:1.4 w/w, 0.7:1.3 w/w, 0.8:1.2 w/w, 0.9:1.1 w/w, 1:1 w/w, 1.1:0.9 w/w, 1.2:0.8 w/w, 1.3:0.7 w/w, 1.4:0.6 w/w, 1.5:0.5 w/w, 1.6:0.4 w/w, 1.7:0.3 w/w, 1.8:0.2 w/w, 1.9:0.1 w/w or 2.0:0.1 w/w. In another alternative, the weight ratio of DUR or a pharmaceutically acceptable salt of DUR (e.g., Na salt) and SUL or a pharmaceutically acceptable salt thereof (e.g., Na salt) present in the disclosed fixed dosage compositions is equivalent to a weight ratio of DUR-Na Salt and SUL-Na Salt of 1:1 w/w.

In another alternative, the fixed dosage compositions comprise DUR-Na Salt and SUL-Na Salt and the weight ratio of DUR-Na Salt and SUL-Na Salt present in the disclosed fixed dosage compositions is equivalent to a weight ratio of DUR and SUL ranging from 0.1:2.0 w/w to 2.0:0.1 w/w. In another alternative, the fixed dosage compositions comprise DUR-Na Salt and SUL-Na Salt and the weight ratio of DUR-Na Salt and SUL-Na Salt present in the disclosed fixed dosage compositions is equivalent to a weight ratio of DUR and SUL ranging from 0.2:1.9 w/w to 1.9:0.2 w/w, from 0.2:1.8 w/w to 1.8:0.2 w/w, from 0.3:1.7 w/w to 1.7:0.3 w/w, from 0.4:1.6 w/w to 1.6:0.4 w/w, from 0.5:1.5 w/w to 1.5:0.5 w/w, from 0.6:1.4 w/w to 1.4:0.6 w/w, from 0.7:1.3 w/w to 1.3:0.7 w/w, from 0.8:1.2 w/w to 1.2:0.8 w/w, or from 0.9:1.1 w/w to 1.1:0.9 w/w. In another alternative, the fixed dosage compositions comprise DUR-Na Salt and SUL-Na Salt and the weight ratio of DUR-Na Salt and SUL-Na Salt present in the disclosed fixed dosage compositions is equivalent to a weight ratio of DUR and SUL ranging from 0.5:1.5 w/w to 1.5:0.5 w/w, from 0.7:1.3 w/w to 1.3:0.7 w/w, from 0.8:1.2 w/w to 1.2:0.8 w/w, or from 0.8:1.1 w/w to 1.1:0.8 w/w. In another alternative, the fixed dosage compositions comprise DUR-Na Salt and SUL-Na Salt and the weight ratio of DUR-Na Salt and SUL-Na Salt present in the disclosed fixed dosage compositions is equivalent to a weight ratio of DUR and SUL ranging from 0.9:1.1 w/w to 1.1:0.9 w/w. In another alternative, the fixed dosage compositions comprise DUR-Na Salt and SUL-Na Salt and the weight ratio of DUR-Na Salt and SUL-Na Salt present in the disclosed fixed dosage compositions is equivalent to a weight ratio of DUR and SUL of 0.1:2.0 w/w, 0.1:1.9 w/w, 0.2:1.8 w/w, 0.3:1.7 w/w, 0.4:1.6 w/w, 0.5:1.5 w/w, 0.6:1.4 w/w, 0.7:1.3 w/w, 0.8:1.2 w/w, 0.9:1.1 w/w, 1:1 w/w, 1.1:0.9 w/w, 1.2:0.8 w/w, 1.3:0.7 w/w, 1.4:0.6 w/w, 1.5:0.5 w/w, 1.6:0.4 w/w, 1.7:0.3 w/w, 1.8:0.2 w/w, 1.9:0.1 w/w or 2.0:0.1 w/w. In another alternative, the fixed dosage compositions comprise DUR-Na Salt and SUL-Na Salt and the weight ratio of DUR-Na Salt and SUL-Na Salt present in the disclosed fixed dosage compositions is equivalent to a weight ratio of DUR and SUL of 1:1 w/w.

In another alternative, the fixed dosage compositions comprise DUR-Na Salt and SUL-Na Salt and the weight ratio of DUR-Na Salt and SUL Na-Salt present in the disclosed fixed dosage compositions ranges from 0.1:2.0 w/w to 2.0:0.1 w/w. In another alternative, the fixed dosage compositions comprise DUR-Na Salt and SUL-Na Salt and the weight ratio of DUR-Na Salt and SUL Na-Salt present in the disclosed fixed dosage compositions ranges from 0.2:1.9 w/w to 1.9:0.2 w/w, from 0.2:1.8 w/w to 1.8:0.2 w/w, from 0.3:1.7 w/w to 1.7:0.3 w/w, from 0.4:1.6 w/w to 1.6:0.4 w/w, from 0.5:1.5 w/w to 1.5:0.5 w/w, from 0.6:1.4 w/w to 1.4:0.6 w/w, from 0.7:1.3 w/w to 1.3:0.7 w/w, from 0.8:1.2 w/w to 1.2:0.8 w/w, or from 0.9:1.1 w/w to 1.1:0.9 w/w. In another alternative, the fixed dosage compositions comprise DUR-Na Salt and SUL-Na Salt and the weight ratio of DUR-Na Salt and SUL Na-Salt present in the disclosed fixed dosage compositions ranges from 0.5:1.5 w/w to 1.5:0.5 w/w, from 0.7:1.3 w/w to 1.3:0.7 w/w, from 0.8:1.2 w/w to 1.2:0.8 w/w, or from 0.8:1.1 w/w to 1.1:0.8 w/w. In another alternative, the fixed dosage compositions comprise DUR-Na Salt and SUL-Na Salt and the weight ratio of DUR-Na Salt and SUL Na-Salt present in the disclosed fixed dosage compositions ranges from 0.9:1.1 w/w to 1.1:0.9 w/w. In another alternative, the fixed dosage compositions comprise DUR-Na Salt and SUL-Na Salt and the weight ratio of DUR-Na Salt and SUL Na-Salt present in the disclosed fixed dosage compositions is 0.1:2.0 w/w, 0.1:1.9 w/w, 0.2:1.8 w/w, 0.3:1.7 w/w, 0.4:1.6 w/w, 0.5:1.5 w/w, 0.6:1.4 w/w, 0.7:1.3 w/w, 0.8:1.2 w/w, 0.9:1.1 w/w, 1:1 w/w, 1.1:0.9 w/w, 1.2:0.8 w/w, 1.3:0.7 w/w, 1.4:0.6 w/w, 1.5:0.5 w/w, 1.6:0.4 w/w, 1.7:0.3 w/w, 1.8:0.2 w/w, 1.9:0.1 w/w or 2.0:0.1 w/w. In another alternative, the fixed dosage compositions comprise DUR-Na Salt and SUL-Na Salt and the weight ratio of DUR-Na Salt and SUL Na-Salt present in the disclosed fixed dosage compositions is 1:1 w/w.

The disclosed fixed dosage composition comprising co-lyophilized DUR and SUL, or pharmaceutically acceptable salts thereof, or the split-fill fixed dosage combination may be reconstituted in a pharmaceutically acceptable liquid (e.g., physiological saline, water-for-injection, sodium chloride solution, dextrose, glucose, lactated ringer, invert sugar injection and the like) prior to administration.

Unless otherwise specified (e.g., as in the case of the fixed dosage compositions "consisting of" DUR and SUL), the disclosed fixed dosage compositions may comprise one or more pharmaceutically acceptable excipients, stabilizers, pH adjusting additives, and the like.

Non-co-lyophilized SUL-DUR is currently in clinical development for the treatment of *A. baumannii* complex (ABC) infections as well as for the treatment of urinary tract infection (pyelonephritis). See e.g., US Clinical Trials Identifier NCT03894046 and NCT03445195. Also the European Medicines agency decision P/0499/2020" discloses a powder for solution for injection comprising durlobactam / sulbactam (SUL-DUR) (EMEA-002807-PIP01-20) for the paediatric treatment of infections due to organisms of the *Acinetobacter baumannii-caicoaceticus* complex. Efficacy is also shown in preclinical models of Burkholderia infections, isolates of Enterobacteriaceae, and *P. aeruginosa* infections. See e.g., PCT/US2015/061076.

The references to the methods of treatment by therapy of this description are to be interpreted as references to compounds, pharmaceutical compositions and medicaments of the present invention for use in those methods". In one embodiment, the disclosed fixed dosage compositions are useful in treating bacterial infections such as those caused by Gram-negative bacteria, also referred to as a Gram-negative infection. In one aspect of this embodiment, the Gram-negative infection is an infection resistant to one or more antibiotics. In one aspect of this embodiment, the Gram-negative infection is a multi-drug resistant infection. In certain embodiments, the Gram-negative bacterium is *Acinetobacter* spp.. In certain embodiments, the Gram-negative bacterium is *Acinetobacter* spp., such as *Acinetobacter baumannii.* In certain embodiments, the Gram-negative bacterium is *Burkholderia* spp.. In certain embodiments, the Gram-negative bacterium is *Burkholderia pseudomallei*. In certain embodiments, the Gram-negative bacterium is *Pseudomonas aeruginosa*. In certain embodiments, the Gram-negative bacterium is Enterobacteriaceae. In any of these embodiments, the Gram-negative infection arises from a pathogen or pathogen expressing one or more β-lactamase. In any of these embodiments, the Gram-negative infection arises from a pathogen or pathogen expressing one or more Class A, Class C and/or Class D β-lactamase. In any of these embodiments, the Gram-negative infection arises from a pathogen or pathogen expressing one or more Class A β-lactamase. In any of these embodiments, the Gram-negative infection arises from a pathogen or pathogen expressing one or more Class C β-lactamase. In any of these embodiments, the Gram-negative infection arises from a pathogen or pathogen expressing one or more Class D β-lactamase.

An infection caused by "Enterobacteriaceae" refers to any of the Gram-negative bacteria in this family of bacteria which includes, but is not limited to, species such as *Salmonella* spp., *Escherichia coli*, *Yersinia pestis*, *Klebsiella* spp., *Shigella* spp., *Proteus* spp., *Enterobacter* spp., *Serratia* spp., and *Citrobacter* spp.. Thus, treatment of a bacterial infection caused by "Enterobacteriaceae" includes any infection caused by any one or more bacteria which is part of this family. In one embodiment, a bacterial infection caused by "Enterobacteriaceae" includes bacterial infections which have at least one *Salmonella* spp. pathogen present. In one embodiment, a bacterial infection caused by "Enterobacteriaceae" includes bacterial infections which have at least one *Escherichia coli* pathogen present. In one embodiment, a bacterial infection caused by "Enterobacteriaceae" includes bacterial infections which have at least one *Yersinia pestis* pathogen present. In one embodiment, a bacterial infection caused by "Enterobacteriaceae" includes bacterial infections which have at least one *Klebsiella* spp. pathogen present. In one embodiment, a bacterial infection caused by "Enterobacteriaceae" includes bacterial infections which have at least one *Shigella* spp. pathogen present. In one embodiment, a bacterial infection caused by "Enterobacteriaceae" includes bacterial infections which have at least one *Proteus* spp. pathogen present. In one embodiment, a bacterial infection caused by "Enterobacteriaceae" includes bacterial infections which have at least one *Enterobacter* spp. pathogen present. In one embodiment, a bacterial infection caused by "Enterobacteriaceae" includes bacterial infections which have at least one *Serratia* spp. pathogen present. In one embodiment, a bacterial infection caused by "Enterobacteriaceae" includes bacterial infections which have at least one *Citrobacter* spp. pathogen present.

In certain embodiments, bacterial infection refers to an infection caused by Gram-negative bacteria, wherein the Gram-negative bacterium is Enterobacteriaceae which expresses one or more Class A, Class B, Class C and/or Class D β-lactamase. In one aspect of this embodiment, the Gram-negative bacterium is an Enterobacteriaceae which expresses at least one Class B β-lactamase.

In certain embodiments, the Gram-negative bacterium is *Acinetobacter* spp. which expresses one or more β-lactamases. In one embodiment, the Gram-negative bacterium is *Acinetobacter baumannii* which expresses one or more Class A, Class C and/or Class D β-lactamase. In one embodiment, the Gram-negative bacterium is *Acinetobacter baumannii* which expresses one or more Class A β-lactamase. In one embodiment, the Gram-negative bacterium is *Acinetobacter baumannii* which expresses one or more Class C β-lactamase. In one embodiment, the Gram-negative bacterium is *Acinetobacter baumannii* which expresses one or more Class D β-lactamase. In one embodiment, the Gram-negative bacterium is *Acinetobacter baumannii* which expresses TEM-1 or KPC-2.

In one aspect, bacterial infection may refer to a gynecological infection. In another aspect, bacterial infection may refer to a respiratory tract infection (RTI). In yet another aspect, bacterial infection may refer to a sexually transmitted disease. In yet another aspect, bacterial infection may refer to a urinary tract infection (UTI). In yet another aspect, bacterial infection may refer to a complicated urinary tract infection (cUTI). In yet another aspect, bacterial infection may refer to acute exacerbation of chronic bronchitis (ACEB). In yet another aspect, bacterial infection may refer to acute otitis media. In yet another aspect, bacterial infection may refer to acute sinusitis. In yet another aspect, bacterial infection may refer to an infection caused by drug resistant bacteria. In yet another aspect, bacterial infection may refer to catheter-related sepsis. In yet another aspect, bacterial infection may refer to chancroid. In yet another aspect, bacterial infection may refer to chlamydia. In yet another aspect, bacterial infection may refer to community-acquired pneumonia (CAP). In yet another aspect, bacterial infection may refer to complicated skin and skin structure infection (cSSSI). In yet another aspect, bacterial infection may refer to an acute bacterial skin and skin-structure infection (ABSSSI). In yet another aspect, bacterial infection may refer to uncomplicated skin and skin structure infection (SSSI). In yet another aspect, bacterial infection may refer to endocarditis. In yet another aspect, bacterial infection may refer to febrile neutropenia. In yet another aspect, bacterial infection may refer to gonococcal cervicitis. In yet another aspect, bacterial infection may refer to gonococcal urethritis. In yet another aspect, bacterial infection may refer to hospital-acquired pneumonia (HAP). In yet another aspect, bacterial infection may refer to ventilator-associated pneumonia (VAP). In yet another aspect, bacterial infection may refer to infections in an immuno-compromised host, such as liver abscesses, biliary tract infections and/or bacteremia. In yet another aspect, bacterial infection may refer to bacteremia. In yet another aspect, bacterial infection may refer to osteomyelitis. In yet another aspect, bacterial infection may refer to sepsis. In yet another aspect, bacterial infection may refer to syphilis. In yet another aspect, bacterial infection may refer to an intra-abdominal infection (IAI). In yet another aspect, bacterial infection may refer to pneumonic, septicemic and/or bubonic plague. In yet another aspect, bacterial infection may refer to anthrax. In yet another aspect, bacterial infection may refer to glanders. In yet another aspect, bacterial infection may refer to melioidosis. In yet another aspect, bacterial infection may refer to tularemia.

Also provided are methods of treating a bacterial infection as described herein in a subject in need thereof, comprising administering to the subject a therapeutically effective amount of a provided fixed dose composition.

Also provided are uses of a provided fixed dose composition for treating a bacterial infection as described herein in a subject in need thereof.

Further provided are uses of a provided fixed dose composition in the manufacture of a medicament for treating a bacterial infection as described herein in a subject in need thereof.

The terms "subject" and "patient" may be used interchangeably, and means a mammal in need of treatment, *e.g*., companion animals (e*.g*., dogs, cats, and the like), farm animals (*e.g*., cows, pigs, horses, sheep, goats and the like) and laboratory animals (*e.g*., rats, mice, guinea pigs and the like). Typically, the subject is a human in need of treatment.

As used herein, the term "treating" or 'treatment" refers to obtaining desired pharmacological and/or physiological effect. The effect can be therapeutic, which includes achieving, partially or substantially, one or more of the following results: partially or totally reducing the extent of the disease, disorder or syndrome; ameliorating or improving a clinical symptom or indicator associated with the disorder; or delaying, inhibiting or decreasing the likelihood of the progression of the disease, disorder or syndrome. In one aspect, treatment may also be continued after symptoms have resolved, for example to delay their recurrence. "Treating" or "treatment" also refers to inhibiting delaying rebound of the infection or symptoms of the infection.

A specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. In one aspect, however, the fixed dosage compositions described herein are administered in an "effective amount" or "therapeutically effective amount." This refers to an amount of the fixed dosage that will elicit a biological or medical response of a subject e.g., a dosage of between 0.01 - 100 mg/kg body weight/day.

In certain aspects, the fixed dosage compositions described herein may be administered orally, parenterally, or topically. The term "parenteral" as used herein includes subcutaneous, intravenous, intramuscular, intra-articular, intra-synovial, intrasternal, intrathecal, intrahepatic, intralesional and intracranial injection or infusion techniques. In some aspects, the disclosed fixed dosage compositions are administered intravenously (e.g., following reconstitution in water-for-injection).

The disclosed fixed dosage combinations may be packaged in the form of a pharmaceutical kit optionally together with instructions for use.

### EXEMPLIFICATION

Durlobactam, and its sodium salt, can be synthesized following the procedures for Compound 1 of example 10 in PCT/GB2013/050869. Sulbactam and its pharmaceutically acceptable salts, is commercially available in the form of the combination Unasyn^{®}, Cefina-SB^{®}, Sulperazone^{®}, Sultamicillin^{®} or Bacperazone^{®}. Synthesis of sulbactam is also well-known in the art. See, for example, Volkmann, et al., Efficient Preparation of 6,6-dihalopenicillanic acids. Synthesis of Penicillanic Acid S,S-dioxide (Sulbactam), J. Org. Chem., 47(17):3344-3345 (1982).

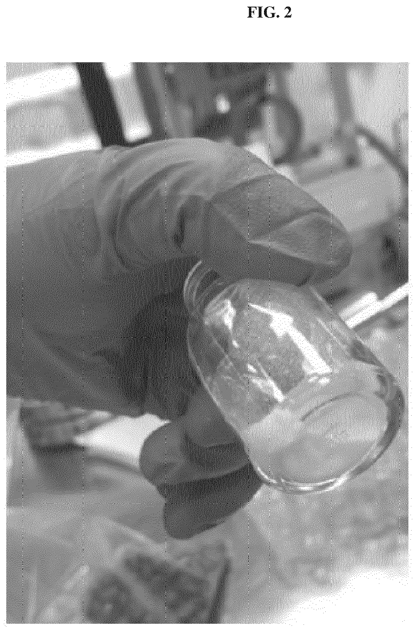

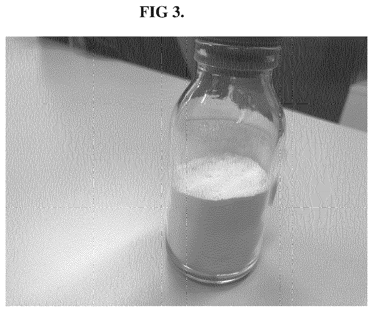

Attempts to develop a commercially feasible fixed-dose combination of durlobactam and sulbactam by dry powder filling both DUR sodium salt and SUL sodium salt (as a 1:1 w/w mixture of each salt form) in glass vials were not successful because of the highly hygroscopic, poor flow properties of DUR sodium salt. This produced significant powder adhesion to the parts of the filling equipment and the dosing chamber and piston of the filling equipment were clogged. See e.g., **FIG. 1****,** showing the DUR sodium salt powder residue from the centralized suction and on the feeding chute observed during machinability trials. See also **FIG. 2** which depicts the appearance of the DUR sodium salt powder filled product. Compare with **FIG 3** which shows the appearance of the bulk powder obtained from co-lyophilizing durlobactam and sulbactam.

Bulk and tapped density analyses of DUR sodium salt were performed according to USP and the compressibility index (CI) and Hausner ratio was calculated. Typically, a material is considered to have poor flowability if the Hausner ratio is greater than 1.25 and/or if the CI is greater than 25. See e.g., Leroy A. Sherrington, Amal Sherrington, in Analytical Profiles of Drug Substances and Excipients, 1998. Here, it was found that the powder had compressibility index and Hausner ratio of 38% and >1.6, indicating an extremely poor flowing material. These experimental values are consistent with the observed powder adhesion.

The flowability issues associated with DUR sodium salt led to the decision to move forward with three separate vials for clinical use. This requires powder filling three separate vials: two containing DUR sodium salt and one containing SUL sodium salt. Each drug product is then separately reconstituted in water-for-injection. Fixed aliquots from each of the three vials are then transferred into an intravenous fluid bag and the resulting combination is then administered to the subject as required. Although promising clinical results were obtained, requiring multi-vial use and multi-processing procedures increases the risk of user error, adds to bulk processing and packaging costs, amplifies waste products, and is overall burdensome.

It has now been found, however, that co-lyophilizing durlobactam sodium salt and sulbactam sodium salt improved flow properties compared to lyophilized durlobactam sodium alone. Initial experiments involved dissolving a 1:1 w/w mixture of durlobactam sodium salt and sulbactam sodium salt in water-for-injection and optimizing the bulk lyophilization conditions (per scale). **Table 1** shows the bulk density, tapped density, and the calculated compressibility index and Hausner ratio for this 1:1 w/w co-lyophilized mixture of durlobactam sodium salt and sulbactam sodium salt.

**Table 1**

| **Test Article** | **Bulk density (g/ml)** | **Tapped density (g/ml)** | **Hausner ratio** | **CI (%)** | |
|---|---|---|---|---|---|
| DUR-Na salt | 0.38 | 0.61 | 1.6 | 38 | |
| 1:1 w/w co-lyophilized SUL-Na/DUR-Na | 0.62 | 0.88 | 1.4 | 30 | Hausner ratio and CI predict poor flowability similar to DUR-Na salt. |

As shown above, there was a slight improvement in the Hausner ratio and compressibility index when going to the co-lyophilized composition. Based on these values and the current literature in the art, one would expect the flowability of the co-lyophilized fixed dosage to prevent formation of a viable fixed dosage combination. This, however, was not the case.

Additionally, the co-lyophilized combination exhibited good stability. To this end, the co-lyophilized mixture was filled into vials and placed on stability at 2-8°C, 25°C/60%RH and 40°C/75%RH. The fixed dose compositions of SUL-Na/DUR-Na, consists of combining both actives co-lyophilized in a single vial. Formulation trials have been conducted to evaluate impact of combining the two actives in a co-lyophilized fixed dose on physical attributes (moisture uptake, particle size distribution, flowability) and chemical stability (assay and impurities). **Tables 2-4** show stability results (3 - 6 months) for the fixed dose compositions when stored at 5 °C, 25 °C/60% RH and 40 °C/75% RH, respectively. The 40 °C/75% RH, represents stress condition and values decrease with concurrent increase in impurity levels. The 5 °C condition, represents the long term storage conditions and the results show almost no change in assay value with little increase in total impurity level. The 25 °C/60% RH condition represents the accelerated storage condition for this product and the total impurity levels are slightly higher than the 5 °C storage condition.

**Table 2 - Stability results of the co-lyophilized fixed dose composition at 5 °C**

| **Months** | **0** | **1** | **2** | **3** | **6** |
|---|---|---|---|---|---|
| Tests | | | | | |
| Appearance of Powder | Off-white powder | Off-white powder | Off-white powder | Off-white powder | Off-white powder |
| Water Content (KF) | 0.9% | 1.0% | 1.1% | 1.0% | 0.9% |
| Durlobactam Assay Recovery | 100.0% | 100.0% | 100.2% | 100.2% | 99.8% |
| Sulbactam Assay Recovery | 100.0% | 100.0% | 100.0% | 100.2% | 100.2% |
| Total impurities | 4.5% | 4.8% | 4.7% | 4.7% | 5.0% |

**Table 3 - Stability results of the co-lyophilized fixed dose composition at 25 °C/60% RH**

| **Months** | **0** | **1** | **2** | **3** | **6** |
|---|---|---|---|---|---|
| Tests | | | | | |
| Appearance of Powder | Off-white powder | Off-white powder | Off-white powder | Off-white powder | Off-white powder |
| Water Content (KF) | 0.9% | 1.0% | 0.9% | 1.0% | 0.9% |
| Durlobactam Assay Recovery | 100.0% | 98.4% | 98.6% | 98.6% | 97.0% |
| Sulbactam Assay Recovery | 100.0% | 99.3% | 100.2% | 100.7% | 100.2% |
| Total impurities | 4.5% | 5.6% | 6.3% | 6.4% | 7.3% |

**Table 4 - Stability results of the co-lyophilized fixed dose composition at 40 °C/75% RH**

| **Months** | **0** | **1** | **2** | **3** |
|---|---|---|---|---|
| Tests | | | | |
| Appearance of Powder | Yellowish powder | Yellowish powder | Yellowish powder | Yellowish powder |
| Water Content (KF) | 0.9% | 1.1% | 1.0% | 1.0% |
| Durlobactam Assay Recovery | 100.0% | 96.0% | 94.9% | 93.5% |
| Sulbactam Assay Recovery | 100.0% | 99.8% | 100.0% | 100.0% |
| Total impurities | 4.5% | 8.0% | 9.4% | 10.6% |

### Alternative Split Fill Approach

A split-fill formulation of durlobactam sodium salt and sulbactam sodium salt was prepared and evaluated for stability. Durlobactam sodium salt was dissolved in water and lyophilized. Sulbactam sodium salt powder and lyophilized durlobactam sodium salt were filled sequentially (lyophilized durlobactam sodium salt powder on top of sulbactam sodium salt powder) in glass vials in presence of inert nitrogen atmosphere, followed by stoppering and crimping. Durlobactam sodium salt and sulbactam sodium salt were present in 1:1 ratio.

**Table 5 - Stability results of the split-fill fixed dose formulation at 5 °C**

| **Months** | **0** | **1** | **2** | **3** | **6** |
|---|---|---|---|---|---|
| **Tests** | | | | | |
| **Appearance of Powder** | Off-white powder | Off-white powder | Off-white powder | Off-white powder | Off-white powder |
| **Water Content (KF)** | 0.6% | 0.6% | 0.6% | 0.6% | 0.6% |
| **Durlobactam (ETX2514) Assay Recovery** | 100.0% | 98.5% | 100.7% | 99.9% | 100.2% |
| **Sulbactam Assay Recovery** | 100.0% | 99.2% | 100.1% | 100.3% | 98.8% |
| **Total impurities** | 4.5% | 4.9% | 4.9% | 4.9% | 5.4% |

**Table 6 - Stability results of the split-fill fixed dose formulation at 25 C/60% RH**

| **Months** | **0** | **1** | **2** | **3** | **6** |
|---|---|---|---|---|---|
| **Tests** | | | | | |
| **Appearance of Powder** | Off-white powder | Off-white powder | Off-white powder | Off-white powder | Off-white powder |
| **Water Content (KF)** | 0.6% | 0.6% | 0.6% | 0.6% | 0.6% |
| **Durlobactam (ETX2514) Assay Recovery** | 100.0% | 98.6% | 98.7% | 98.2% | 96.1% |
| **Sulbactam Assay Recovery** | 100.0% | 100.5% | 99.0% | 100.9% | 99.8% |
| **Total impurities** | 4.5% | 6.1% | 6.6% | 6.7% | 8.0% |

**Table 7 - Stability results of the split-fill fixed dose formulation at 40 C/75% RH**

| **Months** | **0** | **1** | **2** | **3** |
|---|---|---|---|---|
| **Tests** | | | | |
| **Appearance of Powder** | Off-white powder | Pale yellow powder | Pale yellow powder | Yellowish powder |
| **Water Content (KF)** | 0.6% | 0.6% | 0.6% | 0.6% |
| **Durlobactam (ETX2514) Assay Recovery** | 100.0% | 96.0% | 94.9% | 93.5% |
| **Sulbactam Assay Recovery** | 100.0% | 100.0% | 100.1% | 100.8% |
| **Total impurities** | 4.5% | 8.8% | 11.2% | 13.7% |

## Claims

1. A fixed dosage combination comprising durlobactam or a pharmaceutically acceptable salt thereof and sulbactam or a pharmaceutically acceptable salt thereof, wherein the durlobactam or pharmaceutically acceptable salt thereof and sulbactam or pharmaceutically acceptable salt thereof are present as a co-lyophilized mixture.

2. The fixed dosage combination of Claim 1, wherein the fixed dosage combination consists of durlobactam or a pharmaceutically acceptable salt thereof and sulbactam or a pharmaceutically acceptable salt thereof.

3. The fixed dosage combination of Claim 1 or 2, wherein the durlobactam or a pharmaceutically acceptable salt thereof and sulbactam or a pharmaceutically acceptable salt thereof are present in a weight ratio amount ranging from 0.5:1.5 w/w to 1.5:0.5 w/w, from 0.7:1.3 w/w to 1.3:0.7 w/w , from 0.8:1.2 w/w to 1.2:0.8 w/w, or from 0.9:1.1 w/w to 1.1:0.9 w/w; preferably wherein the durlobactam or a pharmaceutically acceptable salt thereof and sulbactam or a pharmaceutically acceptable salt thereof are present in a weight ratio amount ranging from 0.9:1.1 w/w to 1.1:0.9 w/w.

4. The fixed dosage combination of any one of Claims 1 to 3, wherein the durlobactam or a pharmaceutically acceptable salt thereof and sulbactam or a pharmaceutically acceptable salt thereof are present in a weight ratio amount of 1:1 w/w.

5. The fixed dosage combination of any one of Claims 1 to 4, wherein the durlobactam is in the form a sodium salt.

6. The fixed dosage combination of any one of Claims 1 to 5, wherein the sulbactam is in the form of a sodium salt.

7. The fixed dosage combination of Claim 1 or 2, wherein the durlobactam is in the form of a sodium salt and the sulbactam is in the form of a sodium salt, each being present in an amount equivalent to a weight ratio of durlobactam to sulbactam ranging from 0.5:1.5 w/w to 1.5:0.5 w/w, from 0.7:1.3 w/w to 1.3:0.7 w/w, from 0.8:1.2 w/w to 1.2:0.8 w/w, or from 0.9:1.1 w/w to 1.1:0.9 w/w; preferably wherein the durlobactam is in the form of a sodium salt and the sulbactam is in the form of a sodium salt, each being present in an amount equivalent to a weight ratio of durlobactam to sulbactam ranging from 0.9:1.1 w/w to 1.1:0.9 w/w.

8. The fixed dosage combination of any one of Claims 1, 2, and 7, wherein the durlobactam is in the form of a sodium salt and the sulbactam is in the form of a sodium salt, each being present in an amount equivalent to a weight ratio of durlobactam to sulbactam of 1:1 w/w.

9. A fixed dosage combination of any one of Claims 1 to 8 for use in treating a bacterial infection in a subject in need thereof.

10. The fixed dosage combination for use according to Claim 9, wherein the bacterial infection is caused by an Enterobacteriaceae pathogen, an *Acinetobacter* spp. pathogen, a *P. aeruginosa* pathogen, or a *Burkholderia* spp. pathogen.

11. A method of making a fixed dosage solution of durlobactam or a pharmaceutically acceptable salt thereof together with sulbactam or a pharmaceutically acceptable salt thereof, the method comprising co-lyophilizing the durlobactam or pharmaceutically acceptable salt thereof with sulbactam or pharmaceutically acceptable salt thereof to form a co-lyophilized mixture.

12. The method of Claim 11, further comprising reconstituting the co-lyophilized mixture in a pharmaceutically acceptable solvent.

13. The method of Claims 11 or 12, wherein the pharmaceutically acceptable solvent is water.

14. A method of making a fixed dosage solution of durlobactam or a pharmaceutically acceptable salt thereof together with sulbactam or a pharmaceutically acceptable salt thereof, the method comprising mixing lyophilized durlobactam or a pharmaceutically acceptable salt thereof together with sulbactam or a pharmaceutically acceptable salt thereof.

15. A packaged pharmaceutical kit comprising the fixed dosage combination of any one of Claims 1 to 8.

## Patentansprüche

1. Fixdosiskombination, umfassend Durlobactam oder ein pharmazeutisch verträgliches Salz davon und Sulbactam oder ein pharmazeutisch verträgliches Salz davon, wobei das Durlobactam oder das pharmazeutisch verträgliche Salz davon und das Sulbactam oder das pharmazeutisch verträgliche Salz davon als eine co-lyophilisierte Mischung vorhanden sind.

2. Fixdosiskombination nach Anspruch 1, wobei die Fixdosiskombination aus Durlobactam oder einem pharmazeutisch verträglichen Salz davon und Sulbactam oder einem pharmazeutisch verträglichen Salz davon besteht.

3. Fixdosiskombination nach Anspruch 1 oder 2, wobei das Durlobactam oder ein pharmazeutisch verträgliches Salz davon und das Sulbactam oder ein pharmazeutisch verträgliches Salz davon in einer Gewichtsverhältnismenge im Bereich von 0,5:1,5 Gew./Gew. bis 1,5:0,5 Gew./Gew., von 0,7:1,3 Gew./Gew. bis 1,3:0,7 Gew./Gew., von 0,8:1,2 Gew./Gew. bis 1,2:0,8 Gew./Gew. oder von 0,9:1,1 Gew./Gew. bis 1,1:0,9 Gew./Gew. vorhanden sind; vorzugsweise wobei das Durlobactam oder ein pharmazeutisch verträgliches Salz davon und das Sulbactam oder ein pharmazeutisch verträgliches Salz davon in einer Gewichtsverhältnismenge im Bereich von 0,9:1,1 Gew./Gew. bis 1,1:0,9 Gew./Gew. vorhanden sind.

4. Fixdosiskombination nach einem der Ansprüche 1 bis 3, wobei das Durlobactam oder ein pharmazeutisch verträgliches Salz davon und das Sulbactam oder ein pharmazeutisch verträgliches Salz davon in einer Gewichtsverhältnismenge von 1:1 Gew./Gew. vorhanden sind.

5. Fixdosiskombination nach einem der Ansprüche 1 bis 4, wobei das Durlobactam in der Form eines Natriumsalzes vorliegt.

6. Fixdosiskombination nach einem der Ansprüche 1 bis 5, wobei das Sulbactam in der Form eines Natriumsalzes vorliegt.

7. Fixdosiskombination nach Anspruch 1 oder 2, wobei das Durlobactam in der Form eines Natriumsalzes vorliegt und das Sulbactam in der Form eines Natriumsalzes vorliegt, wobei jedes in einer Menge vorhanden ist, die einem Gewichtsverhältnis von Durlobactam zu Sulbactam im Bereich von 0,5:1,5 Gew./Gew. bis 1,5:0,5 Gew./Gew., von 0,7:1,3 Gew./Gew. bis 1,3:0,7 Gew./Gew., von 0,8:1,2 Gew./Gew. bis 1,2:0,8 Gew./Gew. oder von 0,9:1,1 Gew./Gew. bis 1,1:0,9 Gew./Gew. entspricht; vorzugsweise wobei das Durlobactam in der Form eines Natriumsalzes vorliegt und das Sulbactam in der Form eines Natriumsalzes vorliegt, wobei jedes in einer Menge vorhanden ist, die einem Gewichtsverhältnis von Durlobactam zu Sulbactam im Bereich von 0,9:1,1 Gew./Gew. bis 1,1:0,9 Gew./Gew. entspricht.

8. Fixdosiskombination nach einem der Ansprüche 1, 2 und 7, wobei das Durlobactam in der Form eines Natriumsalzes vorliegt und das Sulbactam in der Form eines Natriumsalzes vorliegt, wobei jedes in einer Menge vorhanden ist, die einem Gewichtsverhältnis von Durlobactam zu Sulbactam von 1:1 Gew./Gew. entspricht.

9. Fixdosiskombination nach einem der Ansprüche 1 bis 8 zur Verwendung bei der Behandlung einer bakteriellen Infektion in einem Subjekt, das dieser bedarf.

10. Fixdosiskombination zur Verwendung nach Anspruch 9, wobei die bakterielle Infektion durch ein Enterobacteriaceae-Pathogen, ein *Acinetobacter* spp.-Pathogen, ein *P*. *aeruginosa*-Pathogen oder ein *Burkholderia* spp.-Pathogen verursacht wird.

11. Verfahren zum Herstellen einer Fixdosislösung von Durlobactam oder einem pharmazeutisch verträglichen Salz davon zusammen mit Sulbactam oder einem pharmazeutisch verträglichen Salz davon, wobei das Verfahren das Co-Lyophilisieren des Durlobactams oder des pharmazeutisch verträglichen Salzes davon mit Sulbactam oder dem pharmazeutisch verträglichen Salz davon umfasst, um eine co-lyophilisierte Mischung zu bilden.

12. Verfahren nach Anspruch 11, ferner umfassend das Rekonstituieren der co-lyophilisierten Mischung in einem pharmazeutisch verträglichen Lösungsmittel.

13. Verfahren nach Anspruch 11 oder 12, wobei das pharmazeutisch verträgliche Lösungsmittel Wasser ist.

14. Verfahren zum Herstellen einer Fixdosislösung von Durlobactam oder einem pharmazeutisch verträglichen Salz davon zusammen mit Sulbactam oder einem pharmazeutisch verträglichen Salz davon, wobei das Verfahren das Mischen von lyophilisiertem Durlobactam oder einem pharmazeutisch verträglichen Salz davon zusammen mit Sulbactam oder einem pharmazeutisch verträglichen Salz davon umfasst.

15. Verpacktes pharmazeutisches Kit, umfassend die Fixdosiskombination nach einem der Ansprüche 1 bis 8.

## Revendications

1. Combinaison à dose fixe comprenant du durlobactam ou un sel pharmaceutiquement acceptable de celui-ci et du sulbactam ou un sel pharmaceutiquement acceptable de celui-ci, dans laquelle le durlobactam ou un sel pharmaceutiquement acceptable de celui-ci et le sulbactam ou un sel pharmaceutiquement acceptable de celui-ci sont présents sous la forme d'un mélange co-lyophilisé.

2. Combinaison à dose fixe selon la revendication 1, dans laquelle la combinaison à dose fixe est constituée de durlobactam ou d'un sel pharmaceutiquement acceptable de celui-ci et de sulbactam ou d'un sel pharmaceutiquement acceptable de celui-ci.

3. Combinaison à dosage fixe selon la revendication 1 ou 2, dans laquelle le durlobactam ou un sel pharmaceutiquement acceptable de celui-ci et le sulbactam ou un sel pharmaceutiquement acceptable de celui-ci sont présents dans un rapport pondéral allant de 0,5:1,5 p/p à 1,5:0,5 p/p, de 0,7:1,3 p/p à 1,3:0,7 p/p, de 0,8:1,2 p/p à 1,2:0,8 p/p, ou de 0,9:1,1 p/p à 1,1:0,9 p/p ; de préférence, dans laquelle le durlobactam ou un sel pharmaceutiquement acceptable de celui-ci et le sulbactam ou un sel pharmaceutiquement acceptable de celui-ci sont présents dans un rapport pondéral allant de 0,9:1,1 p/p à 1,1:0,9 p/p.

4. Combinaison à dose fixe selon l'une quelconque des revendications 1 à 3, dans laquelle le durlobactam ou un sel pharmaceutiquement acceptable de celui-ci et le sulbactam ou un sel pharmaceutiquement acceptable de celui-ci sont présents dans un rapport pondéral de 1:1 p/p.

5. Combinaison à dosage fixe selon l'une quelconque des revendications 1 à 4, dans laquelle le durlobactam se présente sous la forme d'un sel de sodium.

6. Combinaison à dosage fixe selon l'une quelconque des revendications 1 à 5, dans laquelle le sulbactam se présente sous la forme d'un sel de sodium.

7. Combinaison à dose fixe selon la revendication 1 ou 2, dans laquelle le durlobactam se présente sous la forme d'un sel de sodium et le sulbactam se présente sous la forme d'un sel de sodium, chacun étant présent en une quantité équivalente à un rapport pondéral durlobactam/sulbactam allant de 0,5:1,5 p/p à 1,5:0,5 p/p, de 0,7:1,3 p/p à 1,3:0,7 p/p, de 0,8:1,2 p/p à 1,2:0,8 p/p, ou de 0,9:1,1 p/p à 1,1:0,9 p/p ; de préférence, dans laquelle le durlobactam se présente sous la forme d'un sel de sodium et le sulbactam se présente sous la forme d'un sel de sodium, chacun étant présent en une quantité équivalente à un rapport pondéral durlobactam/sulbactam allant de 0,9:1,1 p/p à 1,1:0,9 p/p.

8. Combinaison à dose fixe selon l'une quelconque des revendications 1, 2 et 7, dans laquelle le durlobactam se présente sous la forme d'un sel de sodium et le sulbactam se présente sous la forme d'un sel de sodium, chacun étant présent en une quantité équivalente à un rapport pondéral durlobactam/sulbactam de 1:1 p/p.

9. Combinaison à dose fixe selon l'une quelconque des revendications 1 à 8 pour utilisation dans le traitement d'une infection bactérienne chez un sujet qui en a besoin.

10. Combinaison à dose fixe pour utilisation selon la revendication 9, dans laquelle l'infection bactérienne est causée par un pathogène Enterobacteriaceae, un pathogène *Acinetobacter* spp, un pathogène *P. aeruginosa* ou un pathogène *Burkholderia* spp.

11. Procédé de fabrication d'une solution à dose fixe de durlobactam ou d'un sel pharmaceutiquement acceptable de celui-ci conjointement avec du sulbactam ou un sel pharmaceutiquement acceptable de celui-ci, le procédé comprenant la co-lyophilisation du durlobactam ou d'un sel pharmaceutiquement acceptable de celui-ci avec du sulbactam ou un sel pharmaceutiquement acceptable de celui-ci pour former un mélange co-lyophilisé.

12. Procédé selon la revendication 11, comprenant en outre la reconstitution du mélange co-lyophilisé dans un solvant pharmaceutiquement acceptable.

13. Procédé selon les revendications 11 ou 12, dans lequel le solvant pharmaceutiquement acceptable est de l'eau.

14. Procédé de fabrication d'une solution à dose fixe de durlobactam ou d'un sel pharmaceutiquement acceptable de celui-ci conjointement avec du sulbactam ou un sel pharmaceutiquement acceptable de celui-ci, le procédé comprenant le mélange du durlobactam lyophilisé ou d'un sel pharmaceutiquement acceptable de celui-ci avec du sulbactam ou d'un sel pharmaceutiquement acceptable de celui-ci.

15. Kit pharmaceutique conditionné comprenant la combinaison à dosage fixe selon l'une quelconque des revendications 1 à 8.
